Europäisches Patentamt

European Patent Office

Office européen des brevets

(19)

(11) Publication number: **0 228 222 B1**

(12) **EUROPEAN PATENT SPECIFICATION**

(45) Date of publication of the patent specification:
23.05.90

(21) Application number: 86309742.4

(22) Date of filing: 15.12.86

(51) Int. Cl.⁵: **C07C 233/00**, C07C 233/09, C07C 233/57, C07C 381/00, C07F 7/08, A01N 37/18, A01N 37/34, A01N 55/00

(54) Pesticidal dihalovinylalkenylamides.

(30) Priority: 17.12.85 GB 8531073
15.08.86 GB 8619983

(43) Date of publication of application:
08.07.87 Bulletin 87/28

(45) Publication of the grant of the patent:
23.05.90 Bulletin 90/21

(84) Designated Contracting States:
AT BE CH DE ES FR GB GR IT LI LU NL SE

(56) References cited:
DE-A- 2 226 523
DE-A- 2 546 611
FR-A- 2 161 972
FR-A- 2 196 320
GB-A- 2 058 782

Chemical Abstracts, vol. 97, no. 15, October 11, 1982, page 672, abstract 127075m Columbus, Ohio, US
Journal of the Chemical Society, part IV, 1955, pages 4244-4249, London, GB L. Cormbie et al.: "Amides of vegetable origen. Part VII"
Liebigs Annalan der Chemie, no. 5, 1983, pages 1202-1206, Weinheim, DE F. Bohlmann et al.: "Neue N-Isobutylamide aus Heliopsis-Arten"
Chemische Berichte, vol. 100, no. 12, 1967,

(73) Proprietor: THE WELLCOME FOUNDATION LIMITED, 183-193 Euston Road, London NW1 2BP(GB)

(72) Inventor: Blade, Robert John, The Wellcome Research Laboratories, Berkhamsted Hertfordshire(GB)
Inventor: Parkin, Donald, The Wellcome Research Laboratories, Berkhamsted Hertfordshire(GB)
Inventor: Crombie, Leslie, University of Nottingham, University Park Nottingham(GB)
Inventor: Horsham, Mark Andrew, University of Nottingham, University Park Nottingham(GB)

(74) Representative: Rollins, Anthony John et al, Group Patents & Agreements The Wellcome Foundation Ltd Langley Court, Beckenham Kent BR3 3BS(GB)

(56) References cited: (continuation)
pages 3861-3868, Verlag Chemie GmbH, Weinheim, DE F. Bohlmann et al.:"Synthese verschiedener natürlich vorkommender Polyinsäureisobutylamide"

**Description**

This invention relates to chemical compounds having pesticidal, particularly insecticidal, acaricidal and fungicidal, activity.

British Patent Specification No. 1 399 315 (Hoffman La Roche) disclosed certain analogues of juvenile hormone (JH), said to be insecticidal and acaricidal, some of which were unsaturated amides but none of which had a terminal dihalovinyl group remote from the amide group.

German Patent Specification No. 2 226 523 (Sandoz) discloses further JH analogues but, again, there was no disclosure of dihalovinylalkenyl-amides.

One aspect of the present invention provides compounds of Formula (I):

(I) $R^2-(CR^3=CR^4)_a-(CH_2)_b-X-(CH_2)_c(CY=CY^1)_dCONRR^1$

wherein

$a = 1$ or $2$

$b = 0$ to $4$

$R^2$, $R^3$ and $R^4$ are on each occurrence independently selected from hydrogen, cyano, halo, alkoxy, alkoxycarbonyl, cycloalkyl, cycloalkenyl, alkenyl, alkynyl, alkyl (any of which except hydrogen may be optionally substituted by one or more of halo, alkoxy and cyano), acyl or a group $-S(O)_nR^5$ where $R^5$ is alkyl and n is 0 to 2, with the proviso that the terminal substituents ($R^2$, $R^3$) on the terminal vinyl group are halo or trifluoromethyl

X is $-CH_2-$ or $-O-$

$c = 0$ to $10$

$d = 1$ or $2$

each of Y and $Y^1$ is independently selected from hydrogen, ($C_{1-4}$) alkyl and halo-($C_{1-4}$) alkyl; and R and $R^1$ are independently selected from hydrogen, alkyl, amino-alkyl, alkenyl, alkynyl and cycloalkyl, (any of which except hydrogen may be substituted by one or more of alkyl, alkenyl, alkynyl, alkoxy, halo, halo-alkyl and cyano), halo, cyano and dioxalanylalkyl except that R and $R^1$ are not both cyano or both halo.

In the formula above, the terms alkenyl, alkynyl, cycloalkyl and alkoxy refer to groups having from 1 to 10 carbon atoms, preferably 1 to 6.

Preferably, only one of R and $R^1$ is hydrogen. Suitably, R is hydrogen and $R^1$ is alkyl, in which case $C_{1-5}$ alkyl or $C_{1-5}$ alkenyl is preferred, with isobutyl, 1,2-dimethylpropyl, 2,2-dimethylpropyl and 2-methyl-2-propenyl being particularly preferable. 1,2-dimethylpropyl has been found to be associated with good acaricidal activity.

Conveniently, d is 2. The or each olefinic group conjugated to the carbonyl group is preferably of $\underline{E}$ configuration. At least some of Y and $Y^1$ are preferably hydrogen or methyl, most preferably all hydrogen. If any of Y and $Y^1$ are not hydrogen, then any substitution (preferably methyl) is preferably at the 3 position only.

Suitably $b + c = 3$ or $4$: when X is $-CH_2-$, then it is preferred if $b + c = 3$, and when X is $-O-$, then preferably $b + c = 4$. It is generally preferred that c is other than zero.

Advantageously, $a = 1$. Preferably, $R^4$ is hydrogen. Suitably, $R^2$ and $R^3$ are each chloro or fluoro. Most preferably, $R^2$ and $R^3$ are both chloro or both fluoro. Fungicidal activity is particularly associated wit $R^2$ and $R^3$ being fluoro.

A particularly preferred compound is isobutyl (2$\underline{E}$,4$\underline{E}$)-11,11-difluoro-2,4,10-trienamide.

Compounds of Formula (I) may be prepared in any of the following ways:

a) by amidation of the corresponding acid or acid derivative, ie by reaction of a compound of Formula (II) with a compound of Formula (III):

(II) $R^2-(CR^3=CR^4)_a-(CH_2)_b-X-(CH_2)_c(CY=CY^1)_dCOZ^1$

(III) $NHRR^1$

where $Z^1$ is hydroxy, alkoxy, halo or a phosphoroimidate ester group and the other variables are defined above;

b) by reaction of a compound of Formula (IV) with a compound of Formula (V):

(IV) $R^2-(CR^3=CR^4)_a-(CH_2)_b-X-(CH_2)_c(CY=CY^1)_pD^1$

(V) $D^2=CH(CY=CY^1)_qCONRR^1$

wherein one of $D^1$ and $D^2$ is an aldehyde group $-CHO$ and the other is a group $(Z^2)_3P=$ or $(Z^2)_2P(\rightarrow 0)=$, where $Z^2$ is alkyl, alkoxy (preferably ethoxy) or aryl (preferably phenyl), and the sum of p and q is 0 or 1;

c) by β-elimination from a compound of Formula (VII) or (VIII):

(VII) $R^2(CR^3=CR^4)_a(CH_2)_bX(CH_2)_c(CH=CH)_{d-1}CEY-CE^1Y^1CONRR^1$

(VIII) $R^2(CR^3=CR^4)_a(CH_2)_bX(CH_2)_cCEY-CE^1Y^1(CY=CY^1)_{d-1}CONRR^1$

wherein one of E and $E^1$ is hydrogen and the other is a leaving group D ($\rightarrow 0$)L, where D is sulphur or selenium and L is suitable group such as lower alkyl (preferably methyl) or aryl (preferably phenyl);

d) when $R^4$ is hydrogen or alkyl, by reacting a compound of formula (IX) with a comound of Formula (X):

(IX) $D^3(CH_2)_bX(CH_2)_c(CY=CY^1)_dCONRR^1$

(X) $D^4=CR^2R^3$

wherein one of $D^3$ and $D^4$ is a carbonyl group $-C(O)R^4$ and the other is a group $(Z^3)_3P=$, where $Z^3$ is

2

alkyl, alkoxy (preferably ethoxy) or aryl (preferably phenyl) or N(alkyl)$_2$, preferably N(Me)$_2$; R$^2$ and R$^3$ being as defined above.

e) by reacting a compound of Formula (XI) with a compound of Formula (XII)

(XI) A$^1$(CH$_2$)$_c$(CY=CY$^1$)$_d$CONRR$^1$

(XII) R$^2$(CR$^3$=R$^4$)$_a$(CH$_2$)$_b$–Z$^4$

where one of A$^1$ and Z$^4$ is a leaving group such as halide (eg bromide or iodide) and the other is a suitable metal atom such as sodium or lithium or OM where M is hydrogen or a suitable metal such as sodium or lithium;

f) by reaction of a compound of Formula (XIII) with a compound if Formula (XIV):

(XIII) R$^2$–(CR$^3$=CR$^4$)$_a$(CH$_2$)$_b$X(CH$_2$)$_c$–Hal

(XIV) HC≡C(CY=CY$^1$)$_{d-1}$CONRR$^1$

wherein Hal is a halogen atom, followed by reduction of the triple bond, for example by hydrogenation; or

g) by reaction of a compound of Formula (XV) with a compound of Formula (XVI)

(XV) R$^2$–(CR$^3$=CR$^4$)$_a$(CH$_2$)$_b$–X(CH$_2$)$_c$CY=CY$^1$–M$^1$

(XVI) Hal–(CY=CY$^1$)$_{d-1}$CONRR$^1$

wherein Hal is halide (eg bromide or iodide) and M$^1$ is a metal atom or metal group, for example comprising zirconium, aluminium or zinc, eg a bis-(cyclopentadienyl)zirconium chloride group.

Process (a) is normally carried out in an aprotic solvent such as dichloromethane, ether or toluene, optionally in the presence of a tertiary amine such as triethylamine or in the presence of trimethylaluminum (when Z' is alkoxy), but in the absence of water. If the compound of Formula (II) is an acid halide, for example acide chloride, then it may be formed from the corresponding acid by reaction with a suitable reagent such as oxalyl chloride or thionyl chloride. When Z$^1$ is phosphoroimidate group then this is suitably formed from PhOP(O)(NHPh)Cl. The acid function in the compound of Formula (II) may be prepared by hydrolysis of an ester, the ester being prepared by a conventional Wittig or Wadsworth-Emmons reaction, using for example an aldehyde and ethoxycarbonylmethylene triphenylphosphorane or the anion from triethylphosphonocrotonate.

The ester of Formula (II) may, when R$^4$ is hydrogen or alkyl, also be derived by a Wittig reaction using for example an aldehyde or ketone of Formula (XX) with a phosphorane of Formula (X)

(XX) R$^4$C(O)(CH$_2$)$_b$X(CH$_2$)$_c$(CY=CY$^1$)$_d$COOR$^5$

where R$^5$ is alkyl (preferably methyl or ethyl) or aryl (preferably phenyl).

Alternatively, the ester of Formula (II) may be derived by rearrangement and elimination on a compound of formula (XXI)

(XXI) R$^2$–(CR$^3$=CR$^4$)$_a$–(CH$_2$)$_b$–X(CH$_2$)$_{c+2}$CH=C(S(→O)R$^6$)COOR$^5$

wherein R$^5$ is as defined above and R$^6$ is any suitable group, such as phenyl.

The compound of Formula (XXI) may be obtained by reaction of a compound of Formula (XXII) with a compound of Formula (XXIII)

(XXII) R$^2$–(CR$^3$=CR$^4$)$_a$(CH$_2$)$_b$–X(CH$_2$)$_{c+2}$CHO

(XXIII) R$^6$S(→O)CH$_2$COOR$^5$

A further route is for the ester of Formula (II) to be prepared by elimination on a compound of Formula (XXIV):

(XXIV) R$^2$–(CR$^3$=CR$^4$)$_a$–(CH$_2$)$_b$–X(CH$_2$)$_c$–A$^1$–A$^2$–A$^3$–COOR$^5$

wherein R$^5$ is as defined above, one of A$^1$, A$^2$ and A$^3$ is (CH=CH)$_{d-1}$, another of A$^1$, A$^2$ and A$^3$ is –CH$_2$–, and the third of A$^1$, A$^2$ and A$^3$ is –CH(OR$^7$)–, R$^7$ being H or acyl (such as acetyl), and the said –CH$_2$– and –CH(OR$^7$)– groups are adjacent one another. The reaction is preferably carried out in an aromatic solvent, conveniently in the presence of a molybdenum catalyst and a base, such as bistrimethylsilylacetamide. Intermediates of Formula (XXIV) my be obtained by reaction of a suitable aldehyde with a suitable sulphinyl compound, followed by acylation.

Process (b) is suitably carried out in a dry solent, for example tetrahydrofuran (THF), optionally in the presence of a base, and preferably under nitrogen at a low temperature. The Wittig-type reagent of Formula (V) or (VI) may be obtained with lithium diisopropylamide.

Process (c) is normally carried out by heating in an aprotic solvent such as benzene or toluene, preferably in the presence of an acid catalyst, such as paratoluene-sulphonic acid. Process (d) is usually conducted in an aprotic solvent such as THF, diglyme or triglyme at room temperature or below. Process (e) proceeds by heating in an aprotic solvent such as toluene or benzene. Process (f) preferably proceeds by reaction of the compound of Formula (XIV) with a base (such as lithium diisopropylamide) and the compound of Formula (XIII) in an aprotic solvent such as THF. Process (g) is conveniently carried out in an aprotic solvent such as THF, under an inert atmosphere (such as argon) and in the presence of a palladium (0) catalyst, such as bis-(triphenylphosphine) palladium.

The intermediates of Formula (II) to (XX) may be prepared by standard methods. For example, compounds of Formulae (V) and (VI) may be prepared by the reaction of an appropriate phosphine, phosphonate or phosphite with an w-halo amide.

The carbonyl-containing compounds of Formulae (IV), (IX) and (XI) may be prepared, for example, by hydrolysis of a ketal or acetal ring or oxidation of an alcohol, for example using pyridinium chlorochromate or oxalyl chloride/dimethyl sulphoxide. Compounds of Formula (XX) may be prepared, when b = 0, by reaction of the corresponding alcohol with acetic formic anhydride in the presence of a base such as pyridine.

The compounds of Formula I may be used to control arthropods such as insects and acarines, and also fungal pests, such as fungi harmful to crops, stored produce and wood.

The compounds of Formula (I) may be used for such purposes by application of the compounds themselves or in diluted form in known fashion as a dip, spray, lacquer, foam, dust, powder, aqueous suspension, paste, gel, shampoo, grease, combustible solid, vapour emanator (e.g. coil, mat or the like), granule, aerosol, oil suspensions, oil solutions, pressure-pack, impregnated article (such as a plastics ear tag or collar or a strip to treat the air of an enclosed space) or pour on formulation. Dip concentrates are not applied per se, but diluted with water and the animals immersed in a dipping bath containing the dip wash. Sprays may be applied by hand or by means of a spray race or arch. The animal, plant or surface may be saturated with the spray by means of high volume application or superficially coated with the spray by means of light or ultra low volume application. Aqueous suspensions may be applied to the animal in the same manner as sprays or dips. Dusts may be distributed over the animals by means of a powder applicator or incorporated in perforated bags attached to trees or rubbing bars. Pastes, shampoos and greases may be applied manually or distributed over the surface of an inert material against which animals rub and transfer the material to their skins. Pour-on formulations are dispensed as a unit of liquid of small volume on to the backs of animals such that all or most of the liquid is retained on the animals.

The compounds of Formula (I) may be formulated either as formulations ready for use on the animals or as formulations requiring dilution prior to application, but both types of formulation comprise a compound of Formula (I) in intimate admixture with one or more carriers or diluents. The carriers may be liquid, solid or gaseous or comprise mixtures of such substances, and the compound of Formula (I) may be present in a concentration of from 0.025 to 99% w/v depending upon whether the formulation requires further dilution.

Dusts, powder and granules comprise the compound of Formula (I) in intimate admixture with a powdered solid inert carrier for example suitable clays, kaolin, talc, mica, chalk, gypsum, vegetable carriers, starch and diatomaceous earths.

Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil), a wettable powder or a controlled release formulation, such as a microencapsulated formulation. The concentrate preferably comprises a mixture of the active ingredient, with or without an organic solvent and one or more emulsifiers. Solvents may be present within wide limits but preferably in an amount of from 0 to 90% w/v of the composition and may be selected from kerosene, ketones, alcohols, xylene, aromatic naphtha, and other solvents known in the formulating art. The concentration of emulsifiers may be varied within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, and other solvents known in the art.

Wettable powders and emulsifiable concentrates will normally contain from 5 to 95% by weight of the active ingredient, and are diluted, for example with water, before use. Microencapsulated formulations may be made by any known technique, for example coacervation or inter-facial polymerisation.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of formula (I) in intimate admixture with a dispersing agent and one or more surface active agents.

Aqueous suspensions of a compound of formula (I) may comprise a suspension in water together with suspending, stabilizing or other agents. Aqueous solutions may also be formed from acid addition salts of a compound of the formula (I). The suspensions or solutions may be applied per se or in a diluted form in known fashion. Electrostatic spraying techniques may be used with suitable formulations.

Greases (or ointments) may be prepared from vegetable oils, synthetic esters of fatty acids or wool fat together with an inert base such as soft paraffin. A compound of formula (I) is preferably distributed uniformly through the mixture in solution or suspension. Greases may be also be made from emulsifiable concentrates by diluting then with an ointment base.

Pastes and shampoos are also semi-solid preparations in which a compound of formula (I) may be present as an uniform dispersion in suitable base such as soft or liquid paraffin or made on a non-greasy basis with glycerin, mucilage or a suitable soap. As greases, shampoos and pastes are usually applied without further dilution they should contain the appropriate percentage of the compound of formula (I) required for treatment.

Aerosol sprays may be prepared as a simple solution of the active ingredient in the aerosol propellant and co-solvent such as halogenated alkanes and the solvents referred to above, respectively. Pour-on formulations may be made as a solution or suspension of a compound of formula (I) in a liquid medium which also contains a viscous oil to minimise spreading of the formulation on the surface of the animals. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body.

The concentration of the compound of Formula (I) to be applied to a locus (e.g. animal, grain, crop, soil, building etc.) will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the formulation but in general will lie in the range of from 0.0001% to 0.5% except for undiluted formulations such as pour-on formulations which in general will be deposited at a concentration in the range from 0.1 to 20.0% and preferably 0.1 to 10%. For public health usage, a deposited concentration of up to about 5% may be needed. The concentrate may contain up to 90% active ingredient.

Dusts, greases, pastes and aerosol formulations are usually applied in a random fashion as described above and concentrations of 0.001 to 20% w/v of a compound of Formula (I) in the applied formulation my be used.

Bait formulations for, for example, cockroaches will include suitable attractants and/or foodstuffs. The compounds of the invention can be formulated specifically for use on grain or on the exposed surfaces of buildings, or for space spraying.

The compounds may be administered in an animal's feed to combat insect larvae infesting the animal's dung. Any suitable formulation, including microencapsulated material, may be used. The amount of the compound which is administered will vary according to the type and size of animal, and is chosen to provide a suitable concentration of the compounds in the animal's dung. Typically, 0.001 to 100 mg/kg bodyweight, preferably 0.1 to 10 mg/kg, are administered daily, to give concentrations of 0.001 to 1%, preferably 0.01 to 0.1% compound in the dung. The compound will usually be formulated as a concentrate or premix for mixing with a feed supplement, feed concentrate, roughage or the like. Alternatively, the compound may be added to the supply of drinking water. Suitable animals include cattle, pigs, horses, sheep, goats and poultry.

Insect pests include members of the orders Coleoptera (e.g. Anobium, Tribolium, Sitophilus, Diabrotica, Anthonomus, Hylotrupes or Anthrenus spp.), Lepidoptera (e.g. Ephestia, Plutella, Chilo, Heliothis, Spodoptera, Tinea or Tineola spp.), Diptera (e.g. Anopheles, Simulium, Musca, Aedes, Culex, Glossina, Stomoxys, Haematobia, Tabanus, Hydrotaea, Lucilia, Chrysomia, Callitroga, Dermatobia, Hypoderma, Liriomyza, and Melophagus spp.), Phthiraptera (Malophaga e.g. Damalina spp. and Anoplura e.g. Linognathus and Haematopinus spp.), Hemiptera (e.g. Triatoma, Rhodnius, Aphis, Bemisia, Aleurodes, Nilopavata, Nephrotetix or Cimex spp.), Orthoptera (e.g. Schistocerca or Acheta spp.), Dictyoptera (e.g. Blattella, Periplaneta or Blatta spp.), Hymenoptera (e.g. Solenopsis or Monomorium spp.), Isoptera (e.g. Reticulitermes spp.), Siphonaptera (e.g. Ctenocephalides or Pulex spp.), Thysanura (e.g. Lepisma spp.), Dermaptera (e.g. Forficula spp.) and Psocoptera (e.g. Peripsocus spp.). Acarine pests include ticks, e.g. members of the genera Boophilus, Rhipicephalus, Amblyomma, Hyalomma, Ixodes, Haemaphysalis, Dermocentor and Anocentor, and mites and manges such as Tetranychus, Psoroptes, Psorergates, Chorioptes, Demodex, Dermatophagoides, Acarus, Tyrophagus and Glycyphagus spp.

The compounds exhibit killing and/or knockdown activity against adult and/or larval arthropod pests.

Compounds of the invention may be combined with one or more other active ingredients (for example pyrethroids, carbamates and organophosphates) and/or with attractants and the like. Furthermore, it has been found that the activity of the compounds of the invention may be enhanced by the addition of a synergist or potentiator, for example: one of the oxidase inhibitor class of synergists, such as piperonyl butoxide or NIA 16388; a second compound of the invention; or a pyrethroid pesticidal compound. When an oxidase inhibitor synergist is present in a formula of the invention, the ratio of synergist to compound of Formula (I) will be in the range 25:1–1:25 eg about 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin).

It will be understood that what we will claim may comprise:

a) compounds of Formula (I);

b) processes for the preparation of compounds of Formula (I);

c) insecticidal, acaricidal and fungicidal compositions comprising a compound of Formula (I) in admixture with a carrier;

d) processes for the preparation of such pesticidal compositions;

e) methods for the control of insect, acarine or fungal pests comprising the application to the pest or its environment of a compound of Formula (I);

f) synergised pesticidal compositions comprising a compound of Formula (I); and

g) potentiating or non-potentiating mixtures of a compound of Formula (I) and another pesticidal compound;

h) compounds of Formula (II).

The following Examples illustrate, in a non-limiting manner, preferred aspects of the invention. All temperatures are in degrees Celsius.

## Example 1: (2E,4E)–N–ISOBUTYL–11,11–DIFLUOROUNDECA–2,4,10–TRIENAMIDE

Benzyl chloride (20mls, 170 mmol) was added to a mixture of 1,6-hexanediol (75 g, 635 mmol) in toluene (150 mls) containing sodium metal (3.9 g, 170 mmol) and refluxed for 5 hrs. After cooling, the two layers were separated and the upper toluene layer washed with water to remove excess diol. The water layer was back extracted with toluene and the combined fractions dried. Removal of the solvent gave 6-benzyloxyhexan-1-ol (A) as a pale yellow oil which was used without further purification.

Dimethylsulphoxide (7.5 mls, 100 mmol) was added dropwise to a solution of redistilled oxalyl chloride (5.5 mls, 60 mmol) in dry dichloromethane (200 mls) at –60°C under nitrogen. After stirring for 0.5 hr., the monobenzyl protected hexanediol (A) (10 g, 50 mmol) in dry dichloromethane (10 mls) was added and the mixture allowed to warm to –20°C. The mixture was recooled to –70°C and triethylamine (23 mls, 240 mmol) added. After warming to room temperature, water was added (100 mls), the two layers separated, and the organic layer washed with water (2 x 50 mls). The organic layer was dried and evaporated down to give 6 benzyloxyhexanal (B) as a pale yellow oil which was used without further purification.

Sodium chlorodifluoroacetate (12 g, 78 mmol) in warm (60°C) dry diglyme (30 mls) was added via a syringe to a solution of the aldehyde (B) (10 g, 48 mmol) and triphenylphosphine (15.34 g, 58 mmol) in dry diglyme (10 mls) at an oil bath temperature of 160°C. After stirring for 1 hr, the mixture was cooled and the solid (NaCl) filtered off. The diglyme was removed under vacuum and the residue purified by column chromatography (silica, 5:1 hexane:ether) to give 7-benzyloxy-1,1-difluorohept-1-ene(C).

Alternatively, hexamethylphosphorus triamide (12.6 mls, 69 mmol) was added to a solution of dibromodifluoromethane (7.13 g, 34 mmol) in dry diglyme (25 mls) at 0°C. After stirring for 0.5 hr. the aldehyde (B) (6.5 g, 3.2 mmol) in dry diglyme (10 mls) was added and the mixture allowed to warm to room temperature. After 18 hrs., the mixture was worked up with ether, which gave, after removal of solvents, compound (C), an orange oil which was used without further purification.

Trimethyl silyliodide (6 g, 30 mmol) was added to a solution of compound (C) (5.4 g, 22.5 mmol) in dry chloroform (80 mls) under nitrogen, with stirring. After 2 hrs., methanol (100 mls) was added and then the solvents removed under vacuum. The residue was taken up in ether (100 mls), filtered and the filtrate washed successively with aqueous sodium bisulphite (30 mls), aqueous sodium bicarbonate (50 mls) and brine (50 mls). The organic layer was dried and evaporated down and the residue purified by column chromatography give 7,7-difluorohept-6-enol (D). Compound (D) (1 g) was oxidised in an analogous manner to compound (A) to give 7,7-difluorohept-6-enal (E) which was used without further purification.

Triethyl 4-phosphonocrotonate (1.66 g, 6.6 mmol) in dry tetrahydrofuran (THF) (5 mls) was added at –70°C to lithium diisopropylamide (7 mmol) in dry THF (10 mls) under nitrogen. The temperature was allowed to reach –20°C (1 hr) before recooling to –60°C and adding the aldehyde (E) (0.95 g, 6.4 mmol) in dry THF (5 mls). The temperature was allowed to warm slowly to room temperature and then worked up in the standard manner. The crude material was purified by column chromatography (silica, 2:1 hexan:ether) to give (2E,4E) ethyl 11,11-difluoroundeca-2,4,10-trienoate (F) as a pale yellow oil.

The ester (F) (0.6 g, 2.5 mmol) was hydrolysed by refluxing in ethanol (50 mls) containing potassium hydroxide (0.65 g, 12 mmol) for 2 hrs. Work up in the conventional manner gave (2E,4E)-11,11 difluoroundeca-2,4,10 trienoic acid (G) as a pale yellow oil which was carried on to the final stage.

Triethylamine (0.25 mls, 1.8 mmol) was added to the acid (G) (0.38 g, 1.8 mmol) and phenyl N-phenylphosphoramidochloridate (0.48 g, 1.8 mmol) in dry dichloromethane (20 mls) under nitrogen. After stirring for 0.5 hr., triethylamine (0.25 mls, 1.8 mmol) and isobutylamine (0.13 g, 1.8 mmol) in dry dichloromethane (5 mls) were added. The mixture was stirred for 2 hrs. The mixture was washed with water ( 3 x 20 mls), dried and the solvent removed to give an amber residue. This was purified by column chromatography (Silica, 2:1 hexane:ether) to give the title product as a pale yellow solid m.p.

Tlc (silica 1:1 ether:hexane) 1 spot Rf 0.15

Glc 1 peak – Retention time 5 mins.

1H NMR 7.2 (1H),m,H3: 5.9 (3H), m, H2,H4,H5; 4.0 (1H) bm. H10; 3.12 (2H),d of d, N–CH2; 2.3→1.7 and 1.6→1.2 (9H) m, H6→H9, CH of isobutyl; 0.95 (6H),d, isobutyl.

## Example 2: (2E,4E)–N–ISOBUTYL–9–(2,2 DIFLUOROVINYLOXY)–NONA–2,4–DIENAMIDE

(2E,4E)-ethyl 9-hydroxynona-2,4-dienoate (A) was prepared from 1,5-pentanediol by an analogous procedure to that of Example 1.

Acetic formic anhydride (1.3 g, 15 mmol) was added dropwise to the hydroxy ester (A) (2.5 g, 12.6 mmol) in dry dichloromethane (20 mls) containing dry pyridine (2 mls). After stirring under nitrogen for 18 hrs., the mixture was washed with water ( 3 x 20 mls), dried and the solvent removed to give (2E,4E)-ethyl-9-formyloxynona-2,4-dienoate (B) which was carried on to the next stage without further purification.

Compound (B) (3 g) was treated with dibromodifluoromethane by an analogous method to that of Example 1 to give a crude product (2E,4E)-ethyl9-(2,2-difluorovinyloxy)-nona-2,4-dienoate (C) which was further purified by column chromatography (silica, 1:10 hexane:ether)

Isobutylamine (0.23 g, 3.15 mmol) was added dropwise via a syringe to a solution of trimethylaluminium (3.2 mmol) in toluene (10 mls) at –10°C under nitrogen. After stirring for 0.5 hr, the mixture was allowed to warm to room temperature and the ester (C) (0.74 g, 2.85 mmol) in toluene (5 mls) added. The mixture was refluxed for 6 hrs, cooled, and dilute hydrochloric acid (4 mls. 2M) added slowly so as to avoid excess frothing. The toluene layer was washed with water, dried and evaporated down to give crude product. This was further purified by flash column chromatography (silica, 3:1 hexane:ether) to give the title product as an off white solid.

Tlc (silica, 1:1 ether:hexane) 1 spot Rf=0.12; GC (3%OV210, 150→250°C 16°C/min retention time 5.2 mins.

H nmr (dCDCl₃): 7.2 (1H),m, H3, 5.4→6.1(4H), m, H2, H4, H5, NH; 5.6 (1H), dd, olefinic CH; 3.7 (2H), t, H9; 3.15 (2H) d of d, N–CH₂; 2.3→1.4 (7H), m, H6→H8, CH of isobutyl; 0.95 (6H), d, isobutyl.

## Example 3: (2E,4E)–N–ISOBUTYL–11,11–DICHLOROUNDECA–2,4,10–TRIENAMIDE

(2E,4E)-ethyl 10-tetrahydropyranyloxydeca-2,4-dienoate (5.67 g), prepared by an analogous method to that shown in Example 1 from tetrahydropyranyl protected 1,6-hexanediol, was hydrolysed by the method of Example 1 to give (2E,4E)-10-tetrahydropyranyloxydeca-2,4-dienoic acid (A) as a yellow oil. This yielded, by the method of Example 1, (2E,4E)–N–isobutyl 10-tetrahydropyranyloxy-deca-2,4-dienamide (B) as a pale yellow oil. Removal of the protecting group using «Amberlyst 15» resin gave (2E,4E)N–isobutyl 10 hydroxydeca-2,4-dienamide (C) as a white solid after recrystallisation from ether/hexane. («Amberlyst 15» is a trade mark of Rohm and Haas, and is a strongly acidic macroreticular resin available from BDH, Poole, Dorset, UK). Compound (C) (1 g) was then oxidised under the usual conditions to give (2E,4E)–N–isobutyl 9-formylnona-2,4-dienamide (D) as a pale yellow solid.

The aldehyde (D) (0.48 g, 2.0 mmol) and bromotrichloromethane (0.42 g, 2.1 mmol) in dry dichloromethane (2.0 mls) were cooled to –30°C and hexamethylphosphorus triamide (1 g, 6.0 mmol) added dropwise. After stirring at room temperature for 48 hrs, the mixture was washed with water, dilute hydrochloric acid and brine, dried and evapaorated down to give the title product. This was purified by column chromatography (silica, 3:2 hexane:ether) to give the required product as a pale yellow solid. Tlc (silica, 3:2 hexane:ether) 1 spot Rf 0.6; GC (OV210, 250°C) retention time 1.5 mins.

¹H NMR 7.1 (1H), m, H3; 6.18→5.68 (4H), m, H2, H4, H5, H10; 3.4 (1H), t, NH; 3.16 (2H), d of d, N–CH₂; 2.3→1.3 (9H), m, H6→H9, CH of isobutyl; 0.92 (6H), d, 2x Me of isobutyl.

## Example 48: (2E/Z,4E) N–Isobutyl 11,11-difluoro-3-methyl-undeca-2,4,10-trienamide

7,7-Difluorohept-6-ene-1-al (see Example 1) was reacted with triethyl 3-methylphosphonocrotonate to give an ester which was converted to (2E/Z, 4E) N–Isobutyl 11,11-difluoro-3-methyl undeca-2,4,10-trienamide.

## Example 50: (2E/Z,4E) N–Isobutyl 11,11-difluoro-3-fluoromethyl-undeca-2,4,10-trienamide

7,7-Difluorohept-6-ene-1-al was reacted with triethyl-3-fluoromethyl-phosphonocrotonate-LDA to give an ester which was converted to (2E/Z,4E) N–Isobutyl 11,11-difluoro-3-fluoromethyl-undeca-2,4,10-trienamide.

## Example 65: (2E,4E,10Z) N–Isobutyl 11-chloro-11-trifluoromethyl-undeca-2,4,10-trienamide

Deca-10-oxo-2,4-dienoate (14.15 mmol) in dry DMF was treated with acetic anhydride (2.15 ml), trichloroethane (3.36 ml) and activated zinc powder (4.62 g). The mixture was kept at 50°C under nitrogen for 3 hours and worked up in conventional fashion. Chromatography on silica yielded an ester which was reacted with trimethyl aluminium-isobutylamine to give the little compound.

## Examples 5 to 62:

The following tables summarise various examples made by the methods given above or by analogous methods. Unless otherwise stated, all the compounds are (2E,4E) dienamides.

## Table 1

$(R^3)R^2C=CH-CH_2(CH_2)_c(CH=CH)_2CONHR^1$

| Example Number | $R^2$ | $R^3$ | $c$ | $R^1$ | Preparation as Example No. |
|---|---|---|---|---|---|
| 6 | Br | Br | 3 | 1,2-dimethylpropyl | 2 |
| 7 | F | F | 5 | isobutyl | 2 |
| 11 | Cl | Cl | 5 | isobutyl | 3 |
| 12 | F | F | 3 | 2-methylpropenyl | 1 |
| 13 | F | F | 3 | 2-methoxypropyl | 1 |
| 14 | F | F | 3 | 1,2-dimethylpropyl | 2 |
| 52 | F | F | 1 | 1,2-dimethylpropyl | see above |
| 65 | Cl | $CF_3$ | 3 | isobutyl | see above |

## Table 2

$(R^3)R^2C=CH-(CH_2)_bO(CH_2)_c(CH=CH)_2CONHR^1$

| Example Number | $R^2$ | $R^3$ | b | c | $R^1$ | Preparation as Example No. |
|---|---|---|---|---|---|---|
| 21 | Cl | Cl | 1 | 4 | isobutyl | 1 |
| 22 | Cl | Cl | 1 | 4 | 2-methylpropenyl | 1 |
| 23 | Cl | Cl | 1 | 2 | isobutyl | 1 |
| 24 | Cl | Cl | 1 | 2 | 2-methylpropenyl | 1 |
| 25 | F | F | 0 | 5 | 1,2-dimethylpropyl | 2 |
| 26 | F | F | 0 | 5 | isobutyl | 2 |
| 27 | F | F | 0 | 5 | 2-methylpropenyl | 2 |
| 29 | F | F | 0 | 3 | isobutyl | 2 |
| 30 | F | F | 0 | 3 | 1,2-dimethylpropyl | 2 |
| 31 | F | F | 1 | 4 | 1,2-dimethylpropyl | 1 |
| 32 | F | F | 0 | 6 | 1,2-dimethylpropyl | 2 |
| 39 | F | F | 0 | 4 | 1,2-dimethylpropyl | 32 |
| 40 | F | F | 0 | 6 | isobutyl | 32 |

## Table 3

$R^3R^2C=CHCH_2(CH_2)_cCH=CH-CY=CY^1CONHR^1$

| Example Number | $R^2$ | $R^3$ | c | Y | $Y^1$ | $R^1$ | Preparation as Example No. |
|---|---|---|---|---|---|---|---|
| 48 | F | F | 3 | $CH_3$ | H | Isobutyl (49% 2E,4E; 57% 2Z,4E) | |
| 49 | F | F | 3 | $CH_3$ | H | 1,2-dimethylpropyl (40% 2E,4E; 52% 2Z,4E) | 48 |
| 50 | F | F | 3 | $CH_2F$ | H | 1,2-dimethylpropyl (75% 2Z,4E; 25% 2E,4E) | |
| 51 | F | F | 3 | $CH_2F$ | H | 1,2-dimethylpropyl (35% 2Z,4E; 65% 2E,4E) | 50 |

The following compounds of Formula (II) were prepared by methods analogous to that described in Example 1 above.

| Example | Compounds |
|---------|-----------|
| 36 | (2E,4E) ethyl 11,11-difluoroundeca-2,4,10-trienoate |
| 37 | (2E,4E) ethyl 13,13-difluorotrideca-2,4,12-trienoate |
| 38 | (2E,4E) ethyl 9-(3,3-difluoroprop-2-enyloxy)nona-2,4,10-trienoate |
| 46 | (45% 2E,4E; 55% 2Z,4E) ethyl 11,11-difluoro-3-methylundeca-2,4,10-trienoate |
| 47 | (40% 2E,4E; 60% 2Z,4E) ethyl 11,11-difluoro-3-fluoromethylundeca-2,4,10-trienoate |

Biological Examples

A. Lethal Activity against housefly (Musca domestica)

The compounds were administered topically in cellosolve solution in conjunction with a synergist (piperonyl butoxide). The results are given in Table 4.

Table 4

| Compound of Example Number | $LD_{50}$, $\mu$g/fly |
|---------|-----------|
| 1 | <0.5 |
| 2 | <2 |
| 3 | <2 |
| 11 | >6 |
| 12 | <1 |
| 13 | <1.5 |
| 14 | <0.4 |
| 21 | <4 |
| 22 | <3 |
| 23 | <10 |
| 24 | <6 |
| 25 | <6 |
| 26 | <6 |
| 27 | >3 |
| 32 | <2 |

B. Knockdown activity against housefly (Musca domestica)

Knock down (KD) activity against houseflies was gauged by space-spray testing of the compounds in the Kearns and March chamber. The compound was administered in conjunction with a synergist (piperonyl butoxide 1:5 cpd:PB) in dichloromethane/odourless petroleum distillate (OPD). The results are given in Table 5.

Table 5

| Compound of Example No. | Concentration % | $KT_{50}$ (mins) | % Knockdown (at 10 mins) |
|---------|-----------|-----------|-----------|
| 1 | 0.3 | 1.8 | 100 |
| 3 | 0.3 | 4.2 | 99 |
| 12 | 0.3 | 8.5 | 60 |
| 13 | 0.3 | >10 | 32 |
| 14 | 0.3 | 4.8 | 76 |
| 21 | 0.3 | 2.8 | 100 |
| 22 | 0.3 | 5.9 | 94 |

## C1: Activity against Mosquitoes (Culex fatigans)

The compounds were tested in conjunction with piperonyl butoxide in OPD/dichloromethane solution in a wind-tunnel. The results are given in Table 6.

Table 6

| Compound of Example No. | Conc. (%) | KD at 10 mins % | $KT_{50}$ (mins) | Kill % |
|---|---|---|---|---|
| 1 | 0.1 | 80 | 3.6 | 28 |
| 3 | 1 | 92 | 1.9 | 88 |
| 12 | 0.3 | 100 | 1.0 | 96 |
| 13 | 0.3 | 100 | 1.0 | 96 |
| 21 | 0.3 | 86 | 1.0 | 41 |
| 22 | 0.3 | 88 | 1.1 | 26 |
| 23 | 1 | 4 | >10 | 32 |
| 24 | 1 | 4 | >10 | 36 |
| 30 | 1 | 100 | – | 84 |

## C2: Activity against Mosquitoes

Female mosquitoes were confined in glass vials, coated with a deposit of the compound of Example 48, for 30 minutes. They were then removed and held in an untreated container with a supply of sugar-water for 24 hours, at which time mortality was assessed.

| Insect | LC50 (mg m$^{-2}$) |
|---|---|
| Aedes aegypti | 4 |
| Culex quinquefasciatus | 20 |

## D. Lethal Activity against Cockroach (B. germanica)

The compounds were administered topically in cellosolve solution in conjunction with a synergist (piperonyl butoxide). The results are given in Table 7.

Table 7

| Compound of Example Number | $LD_{50}$ µg/Cockroach |
|---|---|
| 1 | <3 |
| 2 | <3 |
| 12 | <5 |
| 14 | <3 |

## E. Antifungal Activity

The compounds were tested on a black filter paper against Coniophora puteana and Coriolus versicolor.
The results are given in Table 8.

Table 8

| Example No. | Coniophora puteana | | Coriolus versicolor | |
|---|---|---|---|---|
| | $IC_{50}$ | $IC_{90}$ | $IC_{50}$ | $IC_{90}$ |
| 1 | 0.8 | 2.9 | 18.5 | >34 |

$IC_{50}$ ($IC_{90}$) is the concentration (µg/cm$^2$) for 50% (90%) inhibition of growth.

EP 0 228 222 B1

## F. Antifungal Activity

The compounds were tested in soft agar against <u>Coniophora puteana</u> and <u>Coriolus versicolor.</u> The results are given in Table 9.

Table 9

| Example No. | Coniophora puteana | | Coriolus versicolor |
|---|---|---|---|
| | $IC_{50}$ | $IC_{90}$ | $IC_{50}$ |
| 1 | 1.5 | 7.0 | 11 |
| 2 | >30 | >30 | 30 |
| 3 | 20.5 | >30 | >30 |
| 12 | 1.9 | 21 | 17 |
| 13 | 5.2 | 24 | >30 |
| 14 | 4.5 | >30 | >30 |
| 26 | >30 | >30 | >30 |
| 29 | >30 | >30 | >30 |
| 30 | >30 | >30 | >30 |
| 37 | 0.6 | 5.5 | 9 |

$IC_{50}$ ($IC_{90}$) is the concentration (ppm) for 50% (90%) inhibition of growth.

## G. Acaricidal Activity

Compounds of Formula (I) were tested against <u>Boophilus microplus</u> (Paquera strain) by injection into females. The results are given in Table 10.

Table 10

| Compound | | Dose (μg/tick) | % Inhibition of Reproduction | % Kill |
|---|---|---|---|---|
| 30 | 10 | 40 | | 0 |

Formulations

1. Emulsifiable Concentrate

| | |
|---|---|
| Compound of Example 1 | 10.00 |
| Ethylan KEO | 20.00 |
| Xylene | 67.50 |
| Butylated Hydroxyanisole | 2.50 |
| | 100.00 |

2. Wettable Powder

| | |
|---|---|
| Compound of Example 1 | 25.0 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

11

### 3. Dust

| Compound of Example 1 | 0.50 |
|---|---|
| Butylated Hydroxyanisole | 0.10 |
| Talc | 99.40 |
| | 100.00 |

### 4. Bait

| Compound of Example 1 | 40.25 |
|---|---|
| Icing Sugar | 99.65 |
| Butylated hydroxy toluene | 0.10 |
| | 100.00 |

### 5. Lacquer

| Compound of Example 1 | 2.5 |
|---|---|
| Resin | 5.0 |
| Butylated Hydroxy anisole | 0.5 |
| High aromatic white spirit | 92.0 |
| | 100.00 |

### 6. Aerosol

| Compound of Example 1 | 0.30 |
|---|---|
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

### 7. Spray

| Compound of Example 1 | 0.1 |
|---|---|
| Butylated Hydroxy anisole | 0.1 |
| Xylene | 10.0 |
| Odourless Kerosene | 89.8 |
| | 100.00 |

### 8. Potentiated Spray

| Compound of Example 1 | 0.1 |
|---|---|
| Permethrin | 0.1 |
| Butylated Hydroxyanisole | 0.1 |
| Xylene | 10.1 |
| Odourless Kerosene | 89.8 |
| | 100.00 |

**Claims for the contracting states BE, CH, DE, ES, FR, GB, GR, IT, LI, LU, NL, SE**

1. A compound of Formula (I):

$$R^2-(CR^3=CR^4)_a-(CH_2)_b-X-(CH_2)_c(CY=CY^1)_dCONRR^1$$

wherein

a = 1 or 2

b = 0 to 4

$R^3$ and $R^4$ are on each occurrence independently selected from hydrogen, cyano, halo, alkoxy, alkoxy, alkoxycarbonyl, cycloalkyl, cycloalkenyl, alkenyl, alkynyl, alkyl (any of which except hydrogen may be optionally substituted by one or more of halo, alkoxy and cyano), acyl or a group $-S(O)_nR^5$ where $R^5$ is alkyl and n is 0 to 2 with the proviso that the terminal substituents ($R^2$, $R^3$) on the terminal vinyl group are halo or trifluoromethyl

X is $-CH_2-$ or $-O-$

c = 0 to 10

d = 1 or 2

each of Y and $Y^1$ is independently selected from hydrogen, ($C_{1-4}$) alkyl and halo–($C_{1-4}$) alkyl; and R and $R^1$ are independently selected from hydrogen, alkyl, amino-alkyl, alkenyl, alkynyl and cycloalkyl, (any of which except hydrogen my be substituted by one or more of alkyl, alkenyl, alkynyl, alkoxy, halo, halo-alkyl and cyano), halo, cyano and dioxalanylalkyl except that R and $R^1$ are not both cyano or both halo.

2. A compound according to claim 1 wherein only one of R and $R^1$ is hydrogen.

3. A compound according to claim 1 or 2 wherein R is hydrogen an $R^1$ is alkyl.

4. A compound according to claim 3 wherein $R^1$ is $C_{1-5}$ alkyl or $C_{1-5}$ alkenyl and d is 2.

5. A compound according to any one of the claims 1 to 4 wherein the or each olefinic group conjugated to the amide carbonyl group is of $\underline{E}$ configuration.

6. A compound according to any one of claims 1 to 5 wherein, when d=2, the carbon atom at the 3 position relative to the amide carbonyl is substituted by methyl or is unsubstituted and the remainder of Y and $Y^1$ are hydrogen.

7. A compound according to any one of claims 1 to 6 wherein $R^2$ and $R^3$ are each chloro or fluoro.

8. Isobutyl ($2\underline{E}$,$4\underline{E}$)-11,11-difluoro-2,4,10-trienamide.

9. A process for preparing a compound according to claim 1:

a) by amidation of the corresponding acid or acid derivative, ie by reaction of a compound of Formula (II) with a compound of Formula (III):

(II) $R^2-(CR^3=CR^4)_a-(CH_2)_b-X-(CH_2)_c(CY=CY^1)_dCOZ^1$

(III) $NHRR^1$

where $Z^1$ is hydroxy, alkoxy, halo or a phosphoroimidate ester group and the other variables are defined above;

b) by reaction of a compound of Formula (IV) with a compound of Formula (V):

(IV) $R^2-(CR^3=CR^4)_a-(CH_2)_b-X-(CH_2)_c(CY=CY^1)_pD^1$

(V) $D^2=CH(CY=CY^1)_qCONRR^1$

wherein one of $D^1$ and $D^2$ is an aldehyde group $-CHO$ and the other is a group $(Z^2)_3P=$ or $(Z^2)_2P(\rightarrow O)=$, where $Z^2$ is alkyl, alkoxy or aryl, and the sum of p and q is 0 or 1;

c) by β-elimination from a compound of Formula (VII) or (VIII):

(VII) $R^2(CR^3=CR^4)_a(CH_2)_bX(CH_2)_c(CH=CH)_{d-1}CEY-CE^1Y^1CONRR^1$

(VIII) $R^2(CR^3=CR^4)_a(CH_2)_bX(CH_2)_cCEY-CE^1Y^1(CY=CY^1)_{d-1}CONRR_1$

wherein one of E and $E^1$ is hydrogen and the other is a leaving group D $(\rightarrow O)L$, where D is sulphur or selenium and L is suitable group such as lower alkyl or aryl;

d) when $R^4$ is hydrogen or alkyl, by reacting a compound of Formula (IX) with a compound of Formula (X):

(IX) $D^3(CH_2)_bX(CH_2)_c(CY=CY^1)_dCONRR^1$

(X) $D^4=CR^2R^3$

wherein one of $D^3$ and $D^4$ is a carbonyl group $-C(O)R^4$ and the other is a group $(Z^3)_3P=$, where $Z^3$ is alkyl, alkoxy or aryl or $N(alkyl)_2$;

$R^2$ and R being as defined above.

e) by reacting a compound of Formula (XI) with a compound of Formula (XII)

(XI) $A^1(CH_2)_c(CY=CY^1)_dCONRR^1$

(XII) $R^2(CR^3=R^4)_a(CH_2)_b-Z^4$

where one of $A^1$ and $Z^4$ is a leaving group such as halide (eg bromide or iodide) and the other is a suitable metal atom or OM where M is hydrogen or a suitable metal;

f) by reaction in an aprotic solvent of a compound of Formula (XIII) with a compound of Formula (XIV):

(XIII) $R^2-(CR^3=CR^4)_a(CH_2)_bX(CH_2)_c-Hal$

(XIV) $HC≡C(CY=CY^1)_{d-1}CONRR^1$

wherein Hal is halogen atom, followed by reduction of the triple bond; or

g) by reaction of a compound of Formula (XV) with a compound of Formula (XVI)

(XV) $R^2-(CR^3=CR^4)_a(CH_2)_b-X(CH_2)_cCY=CY^1-M^1$

(XVI) $Hal-(CY=CY^1)_{d-1}CONRR1$

wherein Hal is halide and $M^1$ is a metal atom or metal group.

10. A pesticidal composition comprising a compound according to any one of claims 1 to 8.

11. A method of combatting insect, acarine or fungal pests by application of a compound according to any one of claims 1 to 8 or a composition according to claim 10.

12. A compound of formula (II) as defined above.

**Claims for the contracting state AT**

1. A process for preparing a compound of Formula (I):

$R^2-(CR^3=CR^4)_a-(CH_2)_b-X-(CH_2)_c(CY=CY^1)_dCONRR^1$

wherein

$a = 1$ or 2

$b = 0$ to 4

$R^3$ and $R^4$ are on each occurrence independently selected from hydrogen, cyano, halo, alkoxy, alkoxycarbonyl, cycloalkyl, cycloalkenyl, alkenyl, alkynyl, alkyl (any of which may be optionally substituted by one or more of halo, alkoxy and cyano), acyl or a group $-S(0)_nR^5$ where $R^5$ is alkyl and $n$ is 0 to 2 with the proviso that the terminal substituents ($R^2$, $R^3$) on the terminal vinyl group are halo or trifluoromethyl

$X$ is $-CH_2-$ or $-O-$

$c = 0$ to 10

$d = 1$ or 2

each of $Y$ and $Y^1$ is independently selected from hydrogen, (C–4) alkyl and halo-($C_{1-4}$) alkyl; and $R$ and $R^1$ are independently selected from hydrogen, alkyl, amino-alkyl, alkenyl, alkynyl and cycloalkyl, (any of which may be substituted by one or more of alkyl, alkenyl, alkynyl, alkoxy, halo, halo-alkyl and cyano), halo, cyano and dioxalanylalkyl except that $R$ and $R^1$ are not both cyano or both halo.

a) by amidation of the corresponding acid or acid derivative, ie by reaction of a compound of Formula (II) with a compound of Formula (III):

(II) $R^2-(CR^3=CR^4)_a-(CH_2)_b-X-(CH_2)_c(CY=CY^1)_dCOZ^1$

(III) $NHRR^1$

where $Z^1$ is hydroxy, alkoxy, halo or a phosphoroimidate ester group and the other variables are defined above;

b) by reaction of a compound of Formula (IV) with a compound of Formula (V):

(IV) $R^2-(CR^3=CR^4)_a-(CH_2)_b-X-(CH_2)_c(CY=CY^1)_pD^1$

(V) $D^2=CH(CY=CY^1)_qCONRR^1$

wherein one of $D^1$ and $D^2$ is an aldehyde group $-CHO$ and the other is a group $(Z^2)_3P=$ or $(Z^2)_2P(\rightarrow O)=$, where $Z^2$ is alkyl, alkoxy or aryl, and the sum of p and q is 0 or 1;

c) by β-elimination from a compound of Formula (VII) or (VIII):

$R^2(CR^3=CR^4)_a(CH_2)_bX(CH_2)_c(CH=CH)_{d-1}CEY-CE^1Y^1CONRR^1$

$R^2(CR^3=CR^4)_a(CH_2)_bX(CH_2)_cCEY-CE^1Y^1(CY=CY^1)_{d-1}CONRR^1$

wherein one of E and $E^1$ is hydrogen and the other is a leaving group D $(\rightarrow O)L$, where D is sulphur or selenium and L is a suitable group;

d) when $R^4$ is hydrogen or alkyl, by reacting a compound of Formula (IX) with a compound of Formula (X):

(IX) $D^3(CH_2)_bX(CH_2)_c(CY=CY^1)_dCONRR^1$

(X) $D^4=CR^2R^3$

wherein one of $D^3$ and $D^4$ is a carbonyl group $-C(O)R^4$ and the other is a group $(Z^3)_3P=$, where $Z^3$ is alkyl, alkoxy or aryl or $N(alkyl)_2$;

$R^2$ and $R^3$ being as defined above.

e) by reacting a compound of Formula (XI) with a compound of Formula (XII)

(XI) $A^1 (CH_2)_c(CY=CY^1)_dCONRR1$

(XII) $R^2(CR^3=R^4)_a(CH_2)_b-Z^4$

where one of $A^1$ and $Z^4$ is a leaving group such as halide and the other is a suitable metal atom such as sodium or lithium or OM where M is hydrogen or a suitable metal;

f) by reaction of a compound of Formula (XIII) with a compound of Formula (XIV):

(XIII) $R^2-(CR^3=CR^4)_a(CH_2)_bX(CH_2)_c-Hal$

(XIV) $HC\equiv C(CY=CY^1)_{d-1}CONRR^1$

wherein Hal is a halogen atom, followed by reduction of the triple bond; or

g) by reaction of a compound of Formula (XV) with a compound of Formula (XVI):

(XV) $R^2-(CR^3=CR^4)_a(CH_2)_bX(CH_2)_cCY=CY^1-M^1$

(XVI) $Hal-(CY=CY^1)_{d-1}CONRR^1$

wherein Hal is halide and $M^1$ is a metal atom or metal group.

2. A process according to claim 1 wherein only one of R and $R^1$ is hydrogen.

3. A process according to claim 1 or 2 wherein R is hydrogen and $R^1$ is alkyl.

4. A process according to claim 3 wherein $R^1$ is $C_{1-5}$ alkyl or $C_{1-5}$ alkenyl and d is 2.

5. A process according to any one of claims 1 to 4 wherein the or each olefinic group conjugated to the amide carbonyl group is of E configuration.

6. A process according to any one of claims 1 to 5 wherein, when d=2, the carbon atom at the 3 position relative to the amide carbonyl is substituted by methyl or is unsubstituted and the remainder of Y and $Y^1$ are hydrogen.

7. A process according to any one of claims 1 to 6 wherein $R^2$ and $R^3$ are each chloro or fluoro.

8. A process according to claim 1 wherein the compound prepared is isobutyl $(2\underline{E},4\underline{E})$-11,11-difluoro-2,4,10-trienamide.

9. A process for preparing a pesticidal formulation containing as active ingredient a compound of Formula (I) which comprises bringing into intimate admixture a compound of Formula (I) as defined in claim 1 and one or more carriers or diluents.

10. A pesticidal formulation comprising a compound of Formula (I) prepared according to any of claims 1 to 8, and one or more carriers or diluents.

11. A method of combatting insect, acarine or fungal pests by applying to the pest or its environment a compound prepared according to any of claims 1 to 8 or a formulation according to claim 11 or 10.

## Patentansprüche für den Vertragsstaat AT

1. Verfahren zum Herstellen einer Verbindung der Formel (I)

$R^2-(CR^3=CR^4)_a-(CH_2)_b-X-(CH_2)_c(CY=CY^1)_dCONRR^1$ (I),

worin

a 1 oder 2 ist,

b Null bis 4 ist,

$R^2$, $R^3$ und $R^4$ an jedem Vorkommen unabhängig ausgewählt sind aus Wasserstoff, Cyano, Halogen, Alkoxy, Alkoxycarbonyl, Cycloalkyl, Cycloalkenyl, Alkenyl, Alkinyl, Alkyl (wovon jedes gegebenenfalls ein- oder mehrfach durch Halogen, Alkoxy und Cyano substituiert sein kann), Acyl oder eine Gruppe $-S(O)_nR^5$ bedeuten, wobei $R^5$ Alkyl darstellt und n Null bis 2 ist, mit der Maßgabe, daß die endständigen Substituenten ($R^2$, $R^3$) an der endständigen Vinylgruppe Halogen oder Trifluormethyl bedeuten,

X $-CH_2-$ oder $-O-$ darstellt,

c Null bis 10 ist,

d 1 oder 2 ist,

Y und $Y^1$ jeweils unabhängig ausgewählt sind aus Wasserstoff, $(C_{1-4})-$Alkyl und Halogen-$(C_{1-4})$alkyl und

R und $R^1$ unabhängig ausgewählt sind aus Wasserstoff, Alkyl, Aminoalkyl, Alkenyl, Alkinyl und Cycloalkyl (wobei jedes hievon ein- oder mehrfach durch Alkyl, Alkenyl, Alkinyl, Alkoxy, Halogen, Halogenalkyl und Cyano substituiert sein kann), Halogen, Cyano und Dioxalanylalkyl, ausgenommen daß R und $R^1$ nicht beide Cyano oder beide Halogen sind,

(a) durch Amidierung der entsprechenden Säure oder des entsprechenden Säurederivats, d.h. durch Umsetzung einer Verbindung der Formel (II)

$R^2-(CR^3=CR^4)_a-(CH_2)_b-X-(CH_2)_c(CY=CY^1)_dCOZ^1$ (II)

mit einer Verbindung der Formel (III)

$NHRR^1$ (III),

worin $Z^1$ Hydroxy, Alkoxy, Halogen oder eine Phosphorimidatestergruppe bedeutet und die anderen Variablen oben definiert sind;

(b) durch Umsetzung einer Verbindung der Formel (IV)

$R^2-(CR^3=CR^4)_a-(CH_2)_b-X-(CH_2)_c(CY=CY^1)_pD^1$ (IV)

mit einer Verbindung der Formel (V)

$D^2=CH(CY=CY^1)_qCONRR^1$ (V),

worin eines von $D^1$ und $D^2$ eine Aldehydgruppe $-CHO$ ist und das andere eine Gruppe $(Z^2)_3P=$ oder $(Z^2)_2P(\rightarrow O)=$ darstellt, wobei $Z^2$ Alkyl, Alkoxy oder Aryl bedeutet und die Summe von p und q Null oder 1 ist;

(c) durch β-Eliminierung von einer Verbindung der Formel (VII)

$R^2(CR^3=CR4)_a(CH_2)_bX(CH_2)_c(CH=CH)_{d-1}CEY-CE^1Y^1CONRR^1$ (VII)

oder (VIII)

$R^2(CR^3=CR^4)_a(CH_2)_bX(CH_2)_cCEY-CE^1Y^1(CY=CY^1)_{d-1}CONRR^1$ (VIII),

worin eines von E und $E^1$ Wasserstoff ist und das andere eine abspaltbare Gruppe $D(\rightarrow O)L$ bedeutet, wobei D Schwefel oder Selen ist und L eine geeignete Gruppe bedeutet;

(d) wenn $R^4$ Wasserstoff oder Alkyl bedeutet, durch Umsetzen einer Verbindung der Formel (IX)

$D^3(CH_2)_bX(CH_2)_c(CY=CY^1)_dCONRR^1$ (IX)

mit einer Verbindung der Formel (X)

$D^4=CR^2R^3$ (X),

worin eines von $D^3$ und $D^4$ eine Carbonylgruppe $-C(O)R^4$ bedeutet und das andere eine Gruppe $(Z^3)_3P=$ darstellt, wobei $Z^3$ Alkyl, Alkoxy, Aryl oder $N(Alkyl)_2$ ist und $R^2$ und $R^3$ wie oben definiert sind;

(e) durch Umsetzen einer Verbindung der Formel (XI)

A$^1$(CH$_2$)$_c$(CY=CY$^1$)$_d$CONRR$^1$ (XI)
mit einer Verbindung der Formel (XII)
R$^2$(CR$^3$=CR$^4$)$_a$(CH$_2$)$_b$–Z$^4$ (XII),

worin eines von A$^1$ und Z$^4$ eine abspaltbare Gruppe, wie ein Halogenid, bedeutet und das andere ein geeignetes Metallatom, wie Natrium oder Lithium, oder OM darstellt, wobei M Wasserstoff oder ein geeignetes Metall ist;
(f) durch Umsetzen einer Verbindung der Formel (XIII)
R$^2$–(CR$^3$=CR$^4$)$_a$(CH$_2$)$_b$X(CH$_2$)$_c$–Hal (XIII)
mit einer Verbindung der Formel (XIV)
HC≡C(CY=CY$^1$)$_{d-1}$CONRR$^1$ (XIV),
worin Hal ein Halogenatom ist, gefolgt von der Reduktion der Dreifachbindung, oder
(g) durch Umsetzen einer Verbindung der Formel (XV)
R$^2$–(CR$^3$=CR$^4$)$_a$(CH$_2$)$_b$X(CH$_2$)$_c$CY=CY$^1$–M$^1$ (XV)
mit einer Verbindung der Formel
Hal–(CY=CY$^1$)$_{d-1}$CONRR$^1$ (XVI),
worin Hal ein Halogenid bedeutet und M$^1$ ein Metallatom oder eine Metallgruppe ist.

2. Verfahren nach Anspruch 1, worin nur eines von R und R$^1$ Wasserstoff ist.

3. Verfahren nach Anspruch 1 oder 2, worin R Wasserstoff bedeutet und R$^1$ Alkyl ist.

4. Verfahren nach Anspruch 3, worin R$^1$ C$_{1-5}$–Alkyl oder C$_{1-5}$–Alkenyl bedeutet und d 2 ist.

5. Verfahren nach einem der Ansprüche 1 bis 4, worin die oder jede an die Amidcarbonylgruppe konjugierte olefinische Gruppe E-Konfiguration aufweist.

6. Verfahren nach einem der Ansprüche 1 bis 5, worin, wenn d 2 ist, das Kohlenstoffatom in Stellung 3 in bezug auf das Amidcarbonyl durch Methyl substituiert oder unsubstituiert ist und der Rest von Y und Y$^1$ Wasserstoff ist.

7. Verfahren nach einem der Ansprüche 1 bis 6, worin R$^2$ und R$^3$ jeweils Chlor oder Fluor bedeuten.

8. Verfahren nach Anspruch 1, worin die hergestellte Verbindung Isobutyl–(2E,4E)–11,11–difluor–2,4,10-trienamid ist.

9. Verfahren zum Herstellen einer pestiziden Formulierung, die als aktiven Bestandteil eine Verbindung der Formel (I) enthält, welches umfaßt, daß man eine Verbindung der Formel (I), wie in Anspruch 1 definiert, und einen oder mehrere Träger oder Verdünnungsmittel in innige Mischung bringt.

10. Pestizide Formulierung umfassend eine Verbindung der Formel (I), hergestellt nach einem der Ansprüche 1 bis 8, und einen oder mehrere Träger oder Verdünnungsmittel.

11. Verfahren zum Bekämpfen von Insekten-, Milben- oder Pilzschädlingen durch Aufbringung einer Verbindung, hergestellt nach einem der Ansprüche 1 bis 8, oder einer Formulierung nach Anspruch 10 auf den Schädling oder dessen Umgebung.

**Patentansprüche für die Vertragsstaaten BE, CH, DE, ES, FR, GB, GR, IT, LI, LU, NL, SE**

1. Verbindung der Formel (I)
R$^2$–(CR$^3$=CR$^4$)$_a$–(CH$_2$)$_b$–X–(CH$_2$)$_c$(CY=CY$^1$)$_d$CONRR$^1$ (I),
worin
a 1 oder 2 ist,
Null bis 4 ist,
R$^2$, R$^3$ und R$^4$ an jedem Vorkommen unabhängig ausgewählt sind aus Wasserstoff, Cyano, Halogen, Alkoxy, Alkoxycarbonyl, Cycloalkyl, Cycloalkenyl, Alkenyl, Alkinyl, Alkyl (wovon jedes gegebenenfalls ein- oder mehrfach durch Halogen, Alkoxy und Cyano substituiert sein kann), Acyl oder eine Gruppe –S(O)nR$^5$ bedeuten, wobei R$^5$ Alkyl darstellt und n Null bis 2 ist, mit der Maßgabe, daß die endständigen Substituenten (R$^2$, R$^3$) an der endständigen Vinylgruppe Halogen oder Trifluormethyl bedeuten,
X –CH$_2$– oder –O– darstellt,
c Null bis 10 ist,
d 1 oder 2 ist,
Y und Y$^1$ jeweils unabhängig ausgewählt sind aus Wasserstoff, (C$_{1-4}$)–Alkyl und Halogen–(C$_{1-4}$)alkyl und
R und R$^1$ unabhängig; ausgewählt sind aus Wasserstoff, Alkyl, Aminoalkyl, Alkenyl, Alkinyl und Cycloalkyl (wobei jedes hievon ein- oder mehrfach durch Alkyl, Alkenyl, Alkinyl, Alkoxy, Halogen, Halogenalkyl und Cyano substituiert sein kann), Halogen, Cyano und Dioxalanylalkyl, ausgenommen daß R und R$^1$ nicht beide Cyano oder beide Halogen sind.

2. Verbindung nach Anspruch 1, worin nur eines von R und R$^1$ Wasserstoff ist.

3. Verbindung nach Anspruch 1 oder 2, worin R Wasserstoff bedeutet und R$^1$ Alkyl ist.

4. Verbindung nach Anspruch 3, worin R$^1$ C$_{1-5}$–Alkyl oder C$_{1-5}$–Alkenyl bedeutet und d 2 ist.

5. Verbindung nach einem der Ansprüche 1 bis 4, worin die oder jede an die Amidcarbonylgruppe konjugierte olefinische Gruppe E-Konfiguration aufweist.

6. Verbindung nach einem der Ansprüche 1 bis 5, worin, wenn d 2 ist, das Kohlenstoffatom in Stellung 3 in bezug auf das Amidcarbonyl durch Methyl substituiert oder unsubstituiert ist und der Rest von Y und $Y^1$ Wasserstoff ist.

7. Verbindung nach einem der Ansprüche 1 bis 6, worin $R^2$ und $R^3$ jeweils Chlor oder Fluor bedeuten.

8. Isobutyl–(2$\underline{E}$,4$\underline{E}$)–11,11–difluor–2,4,10–trienamid.

9. Verfahren zum Herstellen einer Verbindung nach Anspruch 1

(a) durch Amidierung der entsprechenden Säure oder des entsprechenden Säurederivats, d.h. durch Umsetzung einer Verbindung der Formel (II)

$R^2–(CR^3=CR^4)_a–(CH_2)_b–X–(CH_2)_c(CY=CY^1)_dCOZ^1$ (II)

mit einer Verbindung der Formel (III)

$NHRR^1$ (III),

worin $Z^1$ Hydroxy, Alkoxy, Halogen oder eine Phosphorimidatestergruppe bedeutet und die anderen Variablen oben definiert sind;

(b) durch Umsetzung einer Verbindung der Formel (IV)

$R^2–(CR^3=CR^4)_a–(CH_2)_b–X–(CH_2)_c(CY=CY^1)_pD^1$ (IV)

mit einer Verbindung der Formel (V)

$D^2=CH(CY=CY^1)_qCONNR^1$ (V),

worin eines von $D^1$ und $D^2$ eine Aldehydgruppe –CHO ist und das andere eine Gruppe $(Z^2)_3P=$ oder $(Z^2)_2P(\to O)=$ darstellt, wobei $Z^2$ Alkyl, Alkoxy oder Aryl bedeutet und die Summe von p und q Null oder 1 ist;

(c) durch β–Eliminierung von einer Verbindung der Formel (VII)

$R^2(CR^3=CR^4)_a(CH_2)_bX(CH_2)_c(CH=CH)_{d-1}CEY–CE^1Y^1CONNR^1$ (VII)

oder (VIII)

$R^2(CR^3=CR^4)_a(CH_2)_bX(CH_2)_cCEY–CE^1Y^1(CY=CY^1)_{d-1}CONRR^1$ (VIII),

worin eines von E und $E^1$ Wasserstoff ist und das andere eine abspaltbare Gruppe $D(\to O)L$ bedeutet, wobei D Schwefel oder Selen ist und L eine geeignete Gruppe, wie nied.Alkyl oder Aryl, bedeutet;

(d) wenn $R^4$ Wasserstoff oder Alkyl bedeutet, durch Umsetzen einer Verbindung der Formel (IX)

$D^3(CH_2)_bX(CH_2)_c(CY=CY^1)_dCONNR^1$ (IX)

mit einer Verbindung der Formel (X)

$D^4=CR^2R^3$ (X),

worin eines von $D^3$ und $D^4$ eine Carbonylgruppe –C(O)R^4 bedeutet und das andere eine Gruppe $(Z^3)_3P=$ darstellt, wobei $Z^3$ Alkyl, Alkoxy, Aryl oder N(Alkyl)$_2$ ist und $R^2$ und $R^3$ wie oben definiert sind;

(e) durch Umsetzen einer Verbindung der Formel (XI)

$A^1(CH_2)_c(CY=CY^1)_dCONNR^1$ (XI)

mit einer Verbindung der Formel (XII)

$R^2(CR^3=CR^4)_a(CH_2)_b–Z^4$ (XII),

worin eines von $A^1$ und $Z^4$ eine abspaltbare Gruppe, wie ein Halogenid (z.B. Bromid oder Jodid), bedeutet und das andere ein geeignetes Metallatom oder OM darstellt, wobei M Wasserstoff oder ein geeignetes Metall ist;

(f) durch Umsetzen einer Verbindung der Formel (XIII)

$R^2–(CR^3=CR^4)_a(CH_2)_bX(CH_2)_c–Hal$ (XIII)

mit einer Verbindung der Formel (XIV)

$HC\equiv C(CY=CY^1)_{d-1}CONRR^1$ (XIV),

worin Hal ein Halogenatom ist, gefolgt von der Reduktion der Dreifachbindung, oder

(g) durch Umsetzen einer Verbindung der Formel (XV)

$R^2–(CR^3=CR^4)_a(CH_2)_b–X(CH_2)_cCY=CY^1–M^1$ (XV)

mit einer Verbindung der Formel

$Hal–(CY=CY^1)_{d-1}CONRR^1$ (XVI),

worin Hal ein Halogenid bedeutet und $M^1$ ein Metallatom oder eine Metallgruppe ist.

10. Schädlingsbekämpfungsmittel umfassend eine Verbindung nach einem der Ansprüche 1 bis 8.

11. Verfahren zum Bekämpfen von Insekten-, Milben- oder Pilzschädlingen durch Anwendung einer Verbindung nach einem der Ansprüche 1 bis 8 oder eines Mittels nach Anspruch 10.

12. Verbindung der Formel (II), wie oben definiert.

**Revendications pour les Etats contractans BE, CH, DE, ES, FR, GB, GR, IT, LI, LU, NL, SE**

1. Composé de formule (I):

$R^2–(CR^3=CR^4)_a–(CH_2)_b–X–(CH_2)_c(CY=CY^1)_dCONRR^1$ où

a = 1 ou 2

b = 0 à 4

$R^2$, $R^3$ et $R^4$ sont chaque fois choisis indépendamment parmi hydrogène, cyano, halo, alkoxy, alkoxycarbonyle, cycloalkyle, cycloalkényle, alkényle, alkynyle, alkyle (dont l'un quelconque peut être éventuellement substitué par un ou plusieurs d'entre halo, alkoxy et cyano), acyle et un radical $–S(O)_nR^5$, où $R^5$ est

alkyle et n est 0 à 2, avec la restriction que les substituants terminaux ($R^2$, $R^3$) sur le radical vinyle terminal sont halo ou trifluorométhyle;

X est $-CH_2-$ ou $-O-$;

c = 0 à 10;

d = 1 ou 2

Y et $Y^1$ sont choisis chacun indépendamment parmi hydrogène, $(C_{1-4})$-alkyle et halo-$(C_{1-4})$-alkyle; et

R et $R^1$ sont choisis indépendamment parmi hydrogène, alkyle, amino-alkyle, alkényle, alkynyle et cycloalkyle, (dont l'un quelconque peut être substitué par un ou plusieurs d'entre alkyle, alkényle, alkynyle, alkoxy, halo, halo-alkyle et cyano), halo, cyano et dioxalanylalkyle, sauf que R et $R^1$ ne sont pas tous deux cyano ou tous deux halo.

2. Composé suivant la revendication 1, dans lequel un seul d'entre R et $R^1$ est hydrogène.

3. Composé suivant la revendication 1 ou 2, dans lequel R est hydrogène et $R^1$ est alkyle.

4. Composé suivant la revendication 3, dans lequel $R^1$ est $(C_{1-5})$-alkyle ou $(C_{1-5})$-alkényle et d est 2.

5. Composé suivant l'une quelconque des revendications 1 à 4, dans lequel le ou chaque radical oléfinique conjugué au radical carbonyle d'amide est de configuration E̲.

6. Composé suivant l'une quelconque des revendications 1 à 5, dans lequel, lorsque d=2, l'atome de carbone à la position 3 par rapport au radical carbonyle d'amide est substitué par méthyle ou est non substitué et les autres d'entre Y et $Y^1$ sont hydrogène.

7. Composé suivant l'une quelconque des revendications 1 à 6, dans lequel $R^2$ et $R^3$ sont chacun chloro ou fluoro.

8. L'isobutyl-(2E̲,4E̲)-11,11-difluoro-undéca-2,4,10-triénamide.

9. Procédé de préparation d'un composé suivant la revendication 1:

(a) par amidation de l'acide ou dérivé d'acide correspondant, c'est-à-dire par réaction d'un composé de formule (II) avec un composé de formule (III):

(II) $R^2-(CR^3=CR^4)_a-(CH_2)_b-X-(CH_2)_c(CY=CY^1)_dCOZ^1$

(III) $NHRR^1$

où $Z^1$ est hydroxyle, alkoxy ou halo ou bien un radical ester phosphoroimidate et les autres variables sont telles que définies ci-dessus;

(b) par réaction d'un composé de formule (IV) avec un composé de formule (V):

(IV) $R^2-(CR^3=CR^4)_a-(CH_2)_b-X-(CH_2)_c(CY=CY^1)_pD^1$

(V) $D^2=CH(CY=CY^1)_qCONRR^1$

où l'un d'entre $D^1$ et $D^2$ est un radical aldéhyde $-CHO$ et l'autre est un radical $(Z^2)_3P=$ ou $(Z^2)_2P(\rightarrow O)=$, où $Z^2$ est alkyle, alkoxy ou aryle, et la somme de p et q est 0 ou 1;

(c) par élimination en β hors d'un composé de formule

(VII) ou (VIII):

(VII) $R^2(CR^3=CR^4)_a(CH_2)_bX(CH_2)_c(CH=CH)_{d-1}CEY-CE^1Y^1CONRR^1$

(VIII) $R^2(CR^3=CR^4)_a(CH_2)_bX(CH)_cCEY-CE^1Y^1(CY=CY^1)_{d-1}CONRR^1$

où l'un d'entre E et $E^1$ est hydrogène et l'autre est un radical partant $D(\rightarrow O)L$, où D est soufre ou sélénium et L est un radical approprié, comme alkyle inférieur ou aryle;

(d) lorsque $R^4$ est hydrogène ou alkyle, par réaction d'un composé de formule (IX) avec un composé de formule (X):

(IX) $D^3(CH_2)_bX(CH_2)_c(CY=CY^1)_dCONRR^1$

(X) $D^4=CR^2R^3$

où l'un d'entre $D^3$ et $D^4$ est un radical carbonyle $-C(O)R^4$ et l'autre est un radical $(Z^3)_3P=$, où $Z^3$ est alkyle, alkoxy ou aryle ou $N(alkyle)_2$; $R^2$ et $R^3$ étant tels que définis ci-dessus;

(e) par réaction d'un composé de formule (XI) avec un composé de formule (XII)

(XI) $A^1(CH_2)_c(CY=CY^1)_dCONRR^1$

(XII) $R^2(CR^3=R^4)_a(CH_2)_b-Z^4$

où l'un d'entre $A^1$ et $Z^4$ est un radical partant tel qu'halogénure (par exemple bromure ou iodure) et l'autre est un atome de métal approprié ou OM où M est hydrogène ou un métal approprié;

(f) par réaction d'un composé de formule (XIII) avec un composé de formule (XIV):

(XIII) $R^2-(CR^3=CR^4)_a(CH_2)_bX(CH_2)_c-Hal$

(XIV) $HC\equiv C(CY=CY^1)_{d-1}CONRR^1$

où Hal est un atome d'halogène, suivie de la réduction de la triple liaison; ou

(g) par réaction d'un composé de formule (XV) avec un composé de formule (XVI):

(XV) $R^2-(CR^3=CR^4)_a(CH_2)_b-X(CH_2)_cCY=CY^1-M^1$

(XVI) $Hal-(CY=CY^1)_{d-1}CONRR^1$

où Hal est halogénure et $M^1$ est un atome de métal ou radical métallifère.

10. Composition pesticide comprenant un composé suivant l'une quelconque des revendications 1 à 8.

11. Procédé pour combattre les insectes, acariens ou champignons nuisibles, par application d'un composé suivant l'une quelconque des revendications 1 à 8 ou d'une composition suivant la revendication 10.

12. Composé de formule (II) tel que défini ci-dessus.

**Revendications pour l'Etat contractant AT**

1. Procédé de préparation d'un composé de formule I:
$R^2-(CR^3=CR^4)_a-(CH_2)_b-X-(CH_2)_c(CY=CY^1)_dCONRR^1$ où
$a = 1$ ou 2
$b = 0$ à 4
$R^2$, $R^3$ et $R^4$ sont chaque fois choisis indépendamment parmi hydrogène, cyano, halo, alkoxy, alkoxycarbonyle, cycloalkyle, cycloalkényle, alkényle, alkynyle, alkyle (dont l'un quelconque peut être éventuellement substitué par un ou plusieurs d'entre halo, alkoxy et cyano), acyle et un radical $-S(O)_nR^5$, où $R^5$ est alkyle et n est 0 à 2, avec la restriction que les substituants terminaux ($R^2$, $R^3$) sur le radical vinyle terminal sont halo ou trifluorométhyle;
X est $-CH_2-$ ou $-O-$;
$c = 0$ à 10;
$d = 1$ ou 2
Y et $Y^1$ sont choisis chacun indépendamment parmi hydrogène, $(C_{1-4})$-alkyle et halo-$(C_{1-4})$-alkyle; et
R et $R^1$ sont choisis indépendamment parmi hydrogène, alkyle, amino-alkyle, alkényle, alkynyle et cycloalkyle, (dont l'un quelconque peut être substitué par un ou plusieurs d'entre alkyle, alkényle, alkynyle, alkoxy, halo, halo-alkyle et cyano), halo, cyano et dioxalanylalkyle, sauf que R et $R^1$ ne sont pas tous deux cyano ou tous deux halo,

(a) par amidation de l'acide ou dérivé d'acide correspondant, c'est-à-dire par réaction d'un composé de formule (II) avec un composé de formule (III):
(II) $R^2-(CR^3=CR^4)_a-(CH_2)_b-X-(CH_2)_c(CY=CY^1)_dCOZ^1$
(III) $NHRR^1$
où $Z^1$ est hydroxyle, alkoxy ou halo ou bien un radical ester phosphoroimidate et les autres variables sont telles que définies ci-dessus;
(b) par réaction d'un composé de formule (IV) avec un composé de formule (V):
(IV) $R^2-(CR^3=CR^4)_a-(CH_2)_b-X-(CH_2)_c(CY=CY^1)_pD^1$
(V) $D^2=CH(CY=CY^1)_qCONRR^1$
où l'un d'entre $D^1$ et $D^2$ est un radical aldéhyde $-CHO$ et l'autre est un radical $(Z^2)_3P=$ ou $(Z^2)_2P(\rightarrow O)=$, où $Z^2$ est alkyle, alkoxy ou aryle et la somme de p et q est 0 ou 1;
(c) par élimination en β hors d'un composé de formule (VII) ou (VIII):
(VII) $R^2(CR^3=CR^4)_a(CH_2)_bX(CH_2)_c(CH=CH)_{d-1}CEY-CE^1Y^1CONRR^1$ (VIII)
$R^2(CR^3=CR^4)_a(CH_2)_bX(CH_2)_cCEY-CE^1Y^1(CY=CY^1)_{d-1}CONRR^1$
où l'un d'entre E et $E^1$ est hydrogène et l'autre est un radical partant $D(\rightarrow O)L$, où D est soufre ou sélénium et L est un radical approprié;
(d) lorsque $R^4$ est hydrogène ou alkyle, par réaction d'un composé de formule (IX) avec un composé de formule (X):
(IX) $D^3(CH_2)_bX(CH_2)_c(CY=CY^1)_dCONRR^1$
(X) $D^4=CR^2R^3$
où l'un d'entre $D^3$ et $D^4$ est un radical carbonyle $-C(O)R^4$ et l'autre est un radical $(Z^3)_3P=$, où $Z^3$ est alkyle, alkoxy ou aryle ou $N(alkyle)_2$; $R^2$ et $R^3$ étant tels que définis ci-dessus;
(e) par réaction d'un composé de formule (XI) avec un composé de formule (XII)
(XI) $A^1(CH_2)_c(CY=CY^1)_dCONRR^1$
(XII) $R^2(CR^3=R^4)_a(CH_2)_b-Z^4$
où l'un d'entre $A^1$ et $Z^4$ est un radical partant tel qu'halogénure et l'autre est un atome de métal approprié tel que sodium ou lithium, ou OM où M est hydrogène ou un métal approprié;
(f) par réaction d'un composé de formule (XIII) avec un composé de formule (XIV):
(XIII) $R^2-(CR^3=CR^4)_a(CH_2)_bX(CH_2)_c$-Hal
(XIV) $HC≡C(CY=CY^1)_{d-1}CONRR^1$
où Hal est un atome d'halogène, suivie de la réduction de la triple liaison; ou
(g) par réaction d'un composé de formule (XV) avec un composé de formule (XVI):
(XV) $R^2-(CR^3=CR^4)_a(CH_2)_b-X(CH_2)_cCY=CY^1-M^1$
(XVI) $Hal-(CY=CY^1)_{d-1}CONRR^1$
où Hal est halogénure et $M^1$ est un atome de métal ou radical métallifère.

2. Procédé suivant la revendication 1, dans lequel un seul d'entre R et $R^1$ est hydrogène.

3. Procédé suivant la revendication 1 ou 2 dans lequel R est hydrogène et $R^1$ est alkyle.

4. Procédé suivant la revendication 3, dans lequel $R^1$ est $(C_{1-5})$-alkyle ou $(C_{1-5})$-alkényle et d est 2.

5. Procédé suivant l'une quelconque des revendications 1 à 4, dans lequel le ou chaque radical oléfinique conjugué au radical carbonyle d'amide est de configuration E̲.

6. Procédé suivant l'une quelconque des revendications 1 à 5, dans lequel, lorsque d=2, l'atome de carbone à la position 3 par rapport au radical carbonyle d'amide est substitué par méthyle ou est non substitué et les autres d'entre Y et $Y^1$ sont hydrogène.

7. Procédé suivant l'une quelconque des revendications 1 à 6, dans lequel R$^2$ et R$^3$ sont chacun chloro ou fluoro.

8. Procédé suivant la revendication 1, dans lequel le composé préparé est l'isobutyl-(2$\underline{E}$,4$\underline{E}$)-11,11-difluoro-undéca-2,4,10-triénamide.

9. Procédé de préparation d'une composition pesticide comprenant comme constituant actif un composé de formule (I), qui comprend la mise en mélange intime d'un composé de formule (I) tel que défini dans la revendication 1 et d'un ou plusieurs excipients ou diluants.

10. Composition pesticide comprenant un composé de formule (I) préparé suivant l'une quelconque des revendications 1 à 8 et un ou plusieurs excipients ou diluants.

11. Procédé pour combattre les insectes, acariens et champignons nuisibles par application sur ceux-ci ou leur milieu d'un composé préparé suivant l'une quelconque des revendications 1 à 8 ou d'une composition suivant la revendication 10.